# EUROPEAN PATENT SPECIFICATION

(11) **EP 2 714 890 B1**
(45) Date of publication and mention of the grant of the patent: **16.09.2015**
(21) Application number: 12723449.0
(22) Date of filing: 21.05.2012
(51) Int. Cl.: C12N 1/21, C07K 14/555, C12N 9/02, C12N 9/00

(54) **Recombinant expression of soluble interferon**
Rekombinante Expression von löslichem Interferon
Expression recombinante d'un interféron soluble

(30) Priority: 26.05.2011 EP 11167761
(43) Date of publication of application: 09.04.2014
(73) Proprietor: Richter-Helm BioTec GmbH & Co. KG, 22335 Hamburg (DE)
(72) Inventor: SCHILLING, Ralf, 22453 Hamburg (DE); DIEDERICH, Bettina, 21220 Seevetal (DE)
(74) Representative: UEXKÜLL & STOLBERG
(86) International application number: PCT/EP2012/059373
(87) International publication number: WO 2012/160027

(56) References cited:
- EP-A1- 2 000 480
- US-A1- 2006 246 541
- M BECKER-HAPAK ET AL: "Role of rpoS in the regulation of glutathione oxidoreductase ( gor) in Escherichia coli", FEMS MICROBIOLOGY LETTERS, vol. 134, no. 1, 1 December 1995 (1995-12-01), pages 39-44, XP055009670, ISSN: 0378-1097, DOI: 10.1016/0378-1097(95)00378-I
- SMIRNOVA G V ET AL: "Effects of Cystine and Hydrogen Peroxide on Glutathione Status and Expression of Antioxidant Genes in Escherichia coli", BIOCHEMISTRY (MOSCOW), KLUWER ACADEMIC PUBLISHERS-PLENUM PUBLISHERS, NE, vol. 70, no. 8, 1 August 2005 (2005-08-01) , pages 926-934, XP019294660, ISSN: 1608-3040
- HATAHET FERAS ET AL: "Disruption of reducing pathways is not essential for efficient disulfide bond formation in the cytoplasm of E. coli", MICROBIAL CELL FACTORIES, vol. 9, 67, 13 September 2010 (2010-09-13) , XP002679010, ISSN: 1475-2859
- BESSETTE P H ET AL: "Efficient folding of proteins with multiple disulfide bonds in the Escherichia coli cytoplasm", PROCEEDINGS OF THE NATIONAL ACADEMY OF SCIENCES, NATIONAL ACADEMY OF SCIENCES, US, vol. 96, no. 24, 23 November 1999 (1999-11-23), pages 13703-13708, XP002233564, ISSN: 0027-8424, DOI: 10.1073/PNAS.96.24.13703

### SEQUENCE LISTING

<110> Richter-Helm BioTech GmbH & Co. KG
<120> RECOMBINANT EXPRESSION OF SOLUBLE INTERFERON
<130> P 87785
<160> 15
<170> PatentIn version 3.3
<210> 1
   <211> 166
   <212> PRT
   <213> Homo sapiens
<400> 1
   Met Cys Asp Leu Pro Gln Thr His Ser Leu Gly Ser Arg Arg Thr Leu
   1               5                   10                  15
   Met Leu Leu Ala Gln Met Arg Lys Ile Ser Leu Phe Ser Cys Leu Lys
               20                  25                  30
   Asp Arg His Asp Phe Gly Phe Pro Gln Glu Glu Phe Gly Asn Gln Phe
           35                  40                  45
   Gln Lys Ala Glu Thr Ile Pro Val Leu His Glu Met Ile Gln Gln Ile
       50                  55                  60
   Phe Asn Leu Phe Ser Thr Lys Asp Ser Ser Ala Ala Trp Asp Glu Thr
   65                  70                  75                  80
   Leu Leu Asp Lys Phe Tyr Thr Glu Leu Tyr Gln Gln Leu Asn Asp Leu
                   85                  90                  95
   Glu Ala Cys Val Ile Gln Gly Val Gly Val Thr Glu Thr Pro Leu Met
               100                 105                 110
   Lys Glu Asp Ser Ile Leu Ala Val Arg Lys Tyr Phe Gln Arg Ile Thr
           115                 120                 125
   Leu Tyr Leu Lys Glu Lys Lys Tyr Ser Pro Cys Ala Trp Glu Val Val
       130                 135                 140
   Arg Ala Glu Ile Met Arg Ser Phe Ser Leu Ser Thr Asn Leu Gln Glu
   145                 150                 155                 160
   Ser Leu Arg Ser Lys Glu
                   165
<210> 2
   <211> 187
   <212> PRT
   <213> Homo sapiens
<400> 2
   Met Thr Asn Lys Cys Leu Leu Gln Ile Ala Leu Leu Leu Cys Phe Ser
   1               5                   10                  15
   Thr Thr Ala Leu Ser Met Ser Tyr Asn Leu Leu Gly Phe Leu Gln Arg
               20                  25                  30
   Ser Ser Asn Phe Gln Cys Gln Lys Leu Leu Trp Gln Leu Asn Gly Arg
           35                  40                  45
   Leu Glu Tyr Cys Leu Lys Asp Arg Met Asn Phe Asp Ile Pro Glu Glu
       50                  55                  60
   Ile Lys Gln Leu Gln Gln Phe Gln Lys Glu Asp Ala Ala Leu Thr Ile
   65                  70                  75                  80
   Tyr Glu Met Leu Gln Asn Ile Phe Ala Ile Phe Arg Gln Asp Ser Ser
                   85                  90                  95
   Ser Thr Gly Trp Asn Glu Thr Ile Val Glu Asn Leu Leu Ala Asn Val
               100                 105                 110
   Tyr His Gln Ile Asn His Leu Lys Thr Val Leu Glu Glu Lys Leu Glu
           115                 120                 125
   Lys Glu Asp Phe Thr Arg Gly Lys Leu Met Ser Ser Leu His Leu Lys
       130                 135                 140
   Arg Tyr Tyr Gly Arg Ile Leu His Tyr Leu Lys Ala Lys Glu Tyr Ser
   145                 150                 155                 160
   His Cys Ala Trp Thr Ile Val Arg Val Glu Ile Leu Arg Asn Phe Tyr
                   165                 170                 175
   Phe Ile Asn Arg Leu Thr Gly Tyr Leu Arg Asn
               180                 185
<210> 3
   <211> 166
   <212> PRT
   <213> Homo sapiens
<400> 3
   Met Lys Tyr Thr Ser Tyr Ile Leu Ala Phe Gln Leu Cys Ile Val Leu
   1               5                   10                  15
   Gly Ser Leu Gly Cys Tyr Cys Gln Asp Pro Tyr Val Lys Glu Ala Glu
               20                  25                  30
   Asn Leu Lys Lys Tyr Phe Asn Ala Gly His Ser Asp Val Ala Asp Asn
           35                  40                  45
   Gly Thr Leu Phe Leu Gly Ile Leu Lys Asn Trp Lys Glu Glu Ser Asp
       50                  55                  60
   Arg Lys Ile Met Gln Ser Gln Ile Val Ser Phe Tyr Phe Lys Leu Phe
   65                  70                  75                  80
   Lys Asn Phe Lys Asp Asp Gln Ser Ile Gln Lys Ser Val Glu Thr Ile
                   85                  90                  95
   Lys Glu Asp Met Asn Val Lys Phe Phe Asn Ser Asn Lys Lys Lys Arg
               100                 105                 110
   Asp Asp Phe Glu Lys Leu Thr Asn Tyr Ser Val Thr Asp Leu Asn Val
           115                 120                 125
   Gln Arg Lys Ala Ile His Glu Leu Ile Gln Val Met Ala Glu Leu Ser
       130                 135                 140
   Pro Ala Ala Lys Thr Gly Lys Arg Lys Arg Ser Gln Met Leu Phe Arg
   145                 150                 155                 160
   Gly Arg Arg Ala Ser Gln
                   165
<210> 4
   <211> 504
   <212> DNA
   <213> Homo sapiens
<400> 4
   atgtgcgatc tgccgcagac ccatagcctg ggcagccgtc gtaccctgat gctgctggcc      60
   cagatgcgta aaattagcct gtttagctgc ctgaaagatc gtcatgattt tggctttccg     120
   caggaagaat ttggcaacca gtttcagaaa gcggaaacca ttccggtgct gcatgaaatg     180
   attcagcaga tcttcaacct gtttagcacc aaagatagca gcgcggcgtg ggatgaaacc     240
   ctgctggata aattctatac cgaactgtat cagcagctga acgatctgga agcgtgcgtg     300
   attcagggcg tgggcgtgac cgaaaccccg ctgatgaaag aagatagcat tctggccgtg     360
   cgtaaatatt ttcagcgcat caccctgtat ctgaaagaaa aaaaatatag cccgtgcgcg     420
   tgggaagtgg tgcgtgcgga aattatgcgt agctttagcc tgagcaccaa cctgcaggaa     480
   agcctgcgta gcaaagaata ataa                                            504
<210> 5
   <211> 1353
   <212> DNA
   <213> Escherichia coli
<400> 5
   atgactaaac actatgatta catcgccatc ggcggcggca gcggcggtat cgcctccatc      60
   aaccgcgcgg ctatgtacgg ccagaaatgt gcgctgattg aagccaaaga gctgggcggc     120
   acctgcgtaa atgttggctg tgtgccgaaa aaagtgatgt ggcacgcggc gcaaatccgt     180
   gaagcgatcc atatgtacgg cccggattat ggttttgata ccactatcaa taaattcaac     240
   tgggaaacgt tgatcgccag ccgtaccgcc tatatcgacc gtattcatac ttcctatgaa     300
   aacgtgctcg gtaaaaataa cgttgatgta atcaaaggct ttgcccgctt cgttgatgcc     360
   aaaacgctgg aggtaaacgg cgaaaccatc acggccgatc atattctgat cgccacaggc     420
   ggtcgtccga gccacccgga tattccgggc gtggaatacg gtattgattc tgatggcttc     480
   ttcgcccttc ctgctttgcc agagcgcgtg gcggttgttg gcgcgggtta catcgccgtt     540
   gagctggcgg gcgtgattaa cggcctcggc gcgaaaacgc atctgtttgt gcgtaaacat     600
   gcgccgctgc gcagcttcga cccgatgatt tccgaaacgc tggtcgaagt gatgaacgcc     660
   gaaggcccgc agctgcacac caacgccatc ccgaaagcgg tagtgaaaaa taccgatggt     720
   agcctgacgc tggagctgga agatggtcgc agtgaaacgg tggattgcct gatttgggcg     780
   attggtcgcg agcctgccaa tgacaacatc aacctggaag ccgctggcgt taaaactaac     840
   gaaaaaggct atatcgtcgt cgataaatat caaaacacca atattgaagg tatttacgcg     900
   gtgggcgata acacgggtgc agtggagctg acaccggtgg cagttgcagc gggtcgccgt     960
   ctctctgaac gcctgtttaa taacaagccg gatgagcatc tggattacag caacattccg    1020
   accgtggtct tcagccatcc gccgattggt actgttggtt taacggaacc gcaggcgcgc    1080
   gagcagtatg gcgacgatca ggtgaaagtg tataaatcct ctttcaccgc gatgtatacc    1140
   gccgtcacca ctcaccgcca gccgtgccgc atgaagctgg tgtgcgttgg atcggaagag    1200
   aagattgtcg gtattcacgg cattggcttt ggtatggacg aaatgttgca gggcttcgcg    1260
   gtggcgctga agatgggggc aaccaaaaaa gacttcgaca ataccgtcgc cattcaccca    1320
   acggcggcag aagagttcgt gacaatgcgt taa                                 1353
<210> 6
   <211> 46
   <212> DNA
   <213> Artificial
<220>
   <223> primer
<400> 6
   gtccgactta tgcccgagaa gatgttgagc aaacttatcg cttatc 46
<210> 7
   <211> 44
   <212> DNA
   <213> Artificial
<220>
   <223> primer
<400> 7
   tgcaaagaga ttcttggcgg agaaaccata attgcatcta ctcg 44
<210> 8
   <211> 36
   <212> DNA
   <213> Artificial
<220>
   <223> primer
<400> 8
   ggaagcgggc ctgcagcgga caccatcgaa tggcgc 36
<210> 9
   <211> 36
   <212> DNA
   <213> Artificial
<220>
   <223> primer
<400> 9
   gggggggggg gtcgacggag tcgtattgat ttggcg 36
<210> 10
   <211> 70
   <212> DNA
   <213> Artificial
<220>
   <223> primer
<400> 10
   tcgctatcat ctacagagaa atccggcgtt gagttcgggt tgctcagcag ctagatgcat 60
   gctcgagcgg 70
<210> 11
   <211> 70
   <212> DNA
   <213> Artificial
<220>
   <223> primer
<400> 11
   gtaaaggctc catcatgcgc ctgggtgaag accgttccat ggatgtggaa ctctgctgaa 60
   gccagttacc 70
<210> 12
   <211> 73
   <212> DNA
   <213> Artificial
<220>
   <223> primer
<400> 12
   aacgtgctcg gtaaaaataa cgttgatgta atcaaaggct ttgcccgctt aattaaccct 60
   cactaaaggg cgg 73
<210> 13
   <211> 74
   <212> DNA
   <213> Artificial
<220>
   <223> primer
<400> 13
   gatcggccgt gatggtttcg ccgtttacct ccagcgtttt ggcatcaacg taatacgact 60
   cactataggg ctcg 74
<210> 14
   <211> 73
   <212> DNA
   <213> Artificial
<220>
   <223> primer
<400> 14
   caccaagttt gaaactgaga tcatttttga tcatatcaac aaggtggatc aattaaccct 60
   cactaaaggg cgg 73
<210> 15
   <211> 74
   <212> DNA
   <213> Artificial
<220>
   <223> primer
<400> 15
   gcaagtgtat tcgccgttat cgccattcag acggaacgga cggttttgca taatacgact     60
   cactataggg ctcg                                                       74

## Description

The present invention generally relates to the field of recombinant gene expression in host cells. In particular, the invention relates to a host cell for the recombinant expression of a mammalian interferon protein, said cell comprising (a) a functional endogenous *trxB* gene; and (b) an inactivated endogenous *gor* gene. The invention further relates to the use of such host cells for the recombinant expression of an interferon protein. The invention also provides a method for the recombinant expression of a soluble interferon in a host cell.

### BACKGROUND OF THE INVENTION

Interferons are a group of glycoproteins that are secreted by mammalian cells in response to different stimuli, such as viral or bacterial infection. Interferons possess various therapeutically useful characteristics, such as immunostimmulating, anti-proliferative, and anti-viral activities. Accordingly, these proteins are widely applied for the treatment of a number of diseases and disorders, including viral infections and cancers, such as multiple myeloma, leukemias, renal-cell carcinoma, and the like. Based on their cellular origin and antigenicity, human interferons are classified into three groups. In humans, alpha interferons are produced by leukocytes, beta interferons are produced by fibroblasts, and gamma interferons are produced by B cells.

Interferons were originally obtained by purifying the proteins from natural sources, e.g., from blood leukocytes and fibroblasts. The advent of recombinant DNA technology has made the production of interferons feasible also on an industrial scale, e.g., by recombinant expression of interferon proteins in bacterial host cells. *Escherichia coli* is one of the most commonly used bacterial host organisms for the recombinant expression of heterologous proteins owing to the ability to grow rapidly to high cell densities, the availability of a vast number of cloning vectors and the ease of introducing such vectors into the cells for subsequent expression. Most of the interferons which are available today for use in human medicine have been produced in *E. coli* expression systems. On the other hand, the expression of heterologous proteins in *E. coli* is known to be associated with certain limitations which render the production of high amounts of pharmaceutically active proteins challenging. A problem often encountered in *E. coli* expression systems is the inability of the host cell to produce considerable amounts of the recombinant proteins in soluble and active form. Instead, most of the protein is stored in form of inclusion bodies, i.e. in aggregates of denatured proteins. The main reason for the inability to create soluble recombinant proteins resides in the fact that the cytoplasm of *E. coli* is a reducing environment which hampers the formation of disulfide bonds. The recovery of active protein from inclusion bodies requires refolding the proteins by use of reducing agents, such as dithiothreitol and β-mercaptoethanol, or denaturants, such as guanidine-HCl and urea, which is often inefficient and associated with unacceptable product losses.

To overcome these drawbacks, host cells were developed in which certain reductase genes were inactivated to create a more oxidative environment that enables disulfide bond formation. These modified host cells have mutations in the genes encoding the thioredoxin reductase *(trxB)* and the glutathione oxidoreductase (*gor*). By virtue of these mutations, disulfide bond formation in the cytoplasm becomes possible and the yield of soluble product is increased. Host cells having a deficiency both in the *trxB* and the *gor* gene are available, for example, under the trade names Origami^{™} (Novagen) or SHuffle^{™} (NEB). It was shown that *trxB⁻*/*gor⁻* Origami^{™} B(DE3) cells (referred to herein as Origami(DE3) in the following; Novagen) can be successfully used for expressing recombinant interferon α2b as described in the published US application 2009/0258394.

Smirnova et al., Biochemistry, 70(8), p926-934,(2005), discloses just like Becker-Hapak et al., (1995), FEMS Microbiology letters, 134(1), p39-44, *E*. *coli* bacteria wherein the endogenous *gor* gene is functionally inactivated and the endogenous *trxB* gene is still active. EP2000480-A1 (Pasteur Institute, 10 december 2008) shows the production of human interferon in an *E*. *coli* host cell wherein both *gor* and *trxB* are inactive and also carries further modifications to allow survival of the cells.

Despite these improvements, there is still a need for more efficient expression systems which can be used for the reliable production of interferon proteins, in particular human interferon proteins. The new expression systems should enable the production of high amounts of soluble interferon proteins while at the same time minimize the portion of denatured protein. Ideally, the expression systems are easy to handle and allow the production of cytokines such as interferons at reduced costs.

### DISCLOSURE OF THE INVENTION

The present invention provides novel host cells which are particularly suitable for the recombinant expression of mammalian interferon proteins. It was found that improved host cells for the expression of interferon proteins can be generated by inactivating the endogenous gor gene of the host cell and at the same time maintaining a functional endogenous *trxB* gene. Unexpectedly, the maintenance of a functional endogenous *trxB* gene in the host cell results in significantly increased yields of recombinant interferon proteins. It may be speculated that the *trxB* gene product is involved in different regulatory pathways that have an impact on expression yields.

By use of the novel host cells for the recombinant production of interferon, high amounts of soluble proteins are obtained without any substantial formation of inclusion bodies. As shown in the below examples, the host cells of the invention produce considerably higher amounts of soluble interferon than corresponding cells having mutations in both the *gor* and *trxB* gene. Thus, the novel host cells constitute a significant improvement over the art, as they simplify the production of interferon proteins and at the same time reduce production costs.

In a first aspect, the present invention thus relates to a host cell which is suitable for the recombinant expression of an interferon protein, said host cell comprising (a) a functional endogenous *trxB* gene; and (b) an inactivated endogenous *gor* gene. Recombinant expression means that expression of the desired protein is effected by a nucleic acid that has been produced by genetic engineering. Preferably, a host cell that has been provided with an expression vector comprising a polynucleotide sequence that encodes an interferon protein is cultured under suitable conditions so that the interferon is expressed. The invention further relates to the use of such host cells for the recombinant expression of an interferon protein.

According to the invention, any cell that is known in the art to be suitable as a host for the expression of heterologous proteins and that has been modified by inactivating the endogenous *gor* gene can be used. The cell can be of eukaryotic or prokaryotic origin. In one embodiment, the host cell is a mammalian cell, e.g. a primary cell or cell line that is derived from a mouse, rat, hamster, dog, or non-human primate. Mammalian cells that have been proven useful for the heterologous expression of proteins include both primary cells (e.g. murine bone marrow cells, haematopoietic stem cells, murine lymphocytes, muscle cells, hepatocytes, and the like) and established cell lines (e.g. Chinese hamster ovary (CHO) cells, monkey kidney cells (COS), baby hamster kidney (BHK) cells, African green monkey kidney (Vero) cells, Madin Darby canine kidney (MDCK) cells, and the like).

In a further embodiment, the host cell of the invention is a yeast cell. For example, yeast cells which have been derived from the species *Saccharomyces cerevisiae, Schizosaccharomyces pombe, Yarrowia lipolytica, Kluyveromyces lactis* and *Pichia pastoris* have been shown to be useful for the expression of interferon proteins (Skoko et al. (2003) Biotechnol. Appl. Biochem. 38(Pt3):257-65). In a still further embodiment, the host cell of the invention can also be derived from insect cells, such as from cells of the insect species *Aedes aegypti, Autographa californica, Bombyx mori, Spodoptera frugiperda,* or *Trichoplusia ni.*

In view of the ease of handling and the rapid cell growth, the use of bacterial host cells is particularly preferred herein. Bacterial cells from numerous species have been used in the art for the expression of heterologous proteins. Commonly used host cells comprise strains of *Escherichia coli, Corynebacterium glutamicum* and *Bacillus subtilis.* In a preferred embodiment, the bacterial host cell is an *E. coli* cell, such as a cell that has been derived from *E. coli* strain BL21.

According to the invention, the host cell contemplated herein for the expression of the interferon proteins has been genetically manipulated to inactivate the endogenous *gor* gene, i.e. the gene encoding the glutathione oxidoreductase native to the host cell. The gene encoding the glutathione oxidoreductase has been described in the literature in several different organisms. As an example, the nucleotide sequence of the *gor* gene of *E. coli* K12 is provided herein as SEQ ID NO:5. Based on the nucleotide sequence of the *gor* gene, the skilled person will readily be able to inactivate said gene by standard genetic methods, such as site-directed mutagenesis. The inactivation of the *gor* gene will be such that no or substantially no biologically active glutathione oxidoreductase enzyme is expressed in the host cell. For the purpose of the present invention, the *gor* gene will be considered as inactivated if the genetic manipulation has decreased the amount of functional glutathione oxidoreductase enzyme by at least 90%, preferably 95% or more compared to the unmodified host cell.

The inactivation may be accomplished by deleting part of or the whole of the coding sequence of the gor gene in the host cell. Alternatively, it is also be possible to introduce a single nucleotide substitution into the coding sequence of the gene, provided that this substitution occurs in a triplet that codes for an essential residue of the oxidoreductase enzyme. One may also insert one or more nucleotides into the coding region so as to alter the reading frame of the *gor* gene. In another preferred embodiment of the invention, the *gor* gene is inactivated by insertion and excision of another polynucleotide, e.g., a gene conferring resistance to an antibiotic which can be used for selection. Methods that can be used for the inactivation of genes in bacterial, mammalian and yeast cells are well known to a person skilled in the art. For example, numerous methods are described in the literature for the inactivation of a bacterial gene. In addition, site-directed mutagenesis kits are commercially available, e.g. the Quick-Change mutagenesis kit of Stratagene, the Transformer mutagenesis kit of Clontech, the GenTaylor mutagenesis system of Invitrogen, the Altered Sites II *in vitro* mutagenesis kit of Promega, and the Red/ET Recombination Kit of Gene Bridges.

It has been found by the inventors that inactivation of the host cell's endogenous *gor* gene is advantageous to obtain high amounts of the recombinantly expressed soluble interferons. Surprisingly, host cells in which only the endogenous *gor* gene has been inactivated produce significantly higher amounts of interferon proteins when compared to corresponding cells of the *trxB⁻*/*gor⁻* genotype (see below Example 6). Therefore, the host cells contemplated by the present invention comprise a functional endogenous *trxB* gene, i.e. a *trxB* gene that is native to the host cell and gives rise to enzymatically active thioredoxin reductase. As a result, the host cell of the invention is capable of producing a native functional thioredoxin reductase enzyme.

It is to be noted that *trxB⁻*/*gor⁻* double knock-out strains are only viable if they also exhibit a mutation in the gene encoding the alkyl hydroperoxide reductase (*ahpC**). See Faulkner et al. (2008), PNAS, 105(18):6735-40. Both the RHB-T7Δgor/trx described herein and the Origami(DE3) strains thus contained an *ahpC** suppressor mutation. In contrast, strains harbouring only an inactivating *gor* mutation are viable without an *ahpC** mutation, since inactivation of the *gor* gene as such does not have any lethal effect. Thus, the RHB-T7Δgor strain prepared in the below Example 2 did not contain an *ahpC** suppressor mutation.

Accordingly, it is preferred herein that the host cells contemplated by the present invention comprise an endogenous, unmodified, functional *ahpC* gene, i.e. an *ahpC* gene that is native to the host cell, does not contain a suppressor mutation, and is capable of producing enzymatically active alkyl hydroperoxide reductase.

To increase the expression level of the interferon protein to be expressed, the host cell of the invention can comprise a RNA polymerase from a bacteriophage, such as the T7 RNA polymerase. The T7 RNA polymerase ensures a strong and controlled expression of a polynucleotide sequence that is contained in an expression vector and preceded by a T7 promoter. The T7 RNA polymerase gene can be provided to the host cell in form of an expression vector. The expression vector that harbours the T7 RNA polymerase gene may be identical to or different from the vector that includes the interferon sequence for expression. It is preferred, however, that the sequence comprising the T7 RNA polymerase gene is inserted into the genome of the host cell, e.g., into the bacterial chromosome. For example, the T7 RNA polymerase gene can be inserted into the genome of an *E*. *coli* host cell, and the polynucleotide encoding the interferon protein, such as IFN-α2a, is expressed from an expression vector that enables expression in *E. coli* cells. A corresponding gene cassette which comprises the T7 RNA polymerase gene can be introduced into the genome of a bacterial host cell, e.g., by homologous recombination as described in the below examples. In addition, *E. coli* host cells which comprise the T7 RNA polymerase gene are also commercially available, for example, from New England Biolabs (Frankfurt, Germany), see for example NEB Cat# C2566, C3016, C3010, C3013, and vectors of the SHUffle series, such as C3026, C3027, C3029 and C3030. Preferably, the T7 RNA polymerase gene is used in combination with an interferon polynucleotide that has been codon-optimized for being expressed in a prokaryotic microorganism, such as an *E. coli* cell.

The host cell that has been manipulated as described above will then be provided with a polynucleotide sequence encoding an exogenous interferon protein, preferably a human interferon gene. The term "exogenous" refers to the fact that the host cell which is selected for the expression of the interferon does not naturally comprise the polynucleotide which encodes the interferon. The polynucleotide can be introduced into the host cell, e.g., by transformation or transfection. Preferably, the polynucleotide sequence encoding the interferon protein to be expressed is included in an expression vector. The choice of the expression vector will mainly depend on the nature of the host cell.

For example, where the host cell is a mammalian cell, such as a CHO or Vero cell, the expression vector may be, for example, a viral or non-viral expression vector useful for introducing an interferon-encoding polynucleotide into the cell for subsequent expression of the interferon protein encoded by said polynucleotide. The expression vector can be an episomal vector, i.e. one that is capable of self-replicating autonomously within the host cell, or an integrating vector, i.e. one which stably incorporates into the genome of the mammalian cell.

A mammalian expression vector will normally comprise a promoter which is functionally linked to the polynucleotide which encodes the interferon. Suitable promoters include, but are not limited to, the cytomegalovirus immediate early promoter (CMV-IE), the Spleen Focus Forming Virus (SFFV) U3 promoter and the adenoviral major late promoter (Ad MLP). As an optional component, the mammalian expression vector may include a suitable enhancer element for increasing the expression level. Examples include the SV40 early gene enhancer (Dijkema et al (1985) EMBO J. 4: 761) and the enhancer of the long terminal repeat (LTR) of Rous Sarcoma Virus (Gorman et al. (1982b) Proc. Natl. Acad. Sci. 79: 6777). The expression vector also optionally comprises transcription termination sequences and polyadenylation sequences for improved expression of interferon protein. Suitable transcription terminator and polyadenylation signals can, for example, be derived from SV40 (Sambrook et al (1989), Molecular Cloning: A Laboratory Manual). Any other element which is known in the art to support efficient expression may be added to the expression vector, such as the Woodchuck hepatitis post-transcriptional regulatory element (wPRE).

In a particularly preferred aspect, the expression vector is a viral expression vector. Viral vectors for use in the present invention typically comprise a viral genome in which a portion of the native sequence has been deleted in order to introduce a heterogeneous polynucleotide without destroying the infectivity of the virus. Due to the specific interaction between virus components and host cell receptors, viral vectors are highly suitable for efficient transfer of genes into target cells. Suitable viral vectors for facilitating gene transfer into a mammalian cell are well known in the art and can be derived from different types of virus, for example, from a retrovirus, adenovirus and adeno-associated virus (AAV), orthomyxovirus, paramyxovirus, papovavirus, picornavirus, or alphavirus. For an overview of the different viral vector systems, see Nienhuis et al., Hematology, Vol. 16: Viruses and Bone Marrow, N.S. Young (ed.), 353-414 (1993).

On the other hand, where the host cell is a bacterial cell, such as an *E. coli* cell, the expression vector will be adapted to protein expression in a prokaryotic cell environment. A vast number of expression vectors have been described for E. *coli* and other bacterial hosts. Examples for vectors suitable for protein expression in *E. coli* cells comprise, for example, the vectors of the pBluescript series, the vectors of the pUC series, e.g. pUC18, pUC19, pBR322, pBR329, pQE70, pQE60, pQE-9, pNH8A, pNH16a, pNH18A, pNH46A, ptrc99a, pKK233-3, pDR540, pRIT5, pLG338, pKC30, pHSG299, pHSG399, pACYC177, pACYC184, pRSF1010, pBW22, and the like. A preferred vector is one that belongs to the pET group of vectors. pET vectors typically comprise a *lacI* gene which codes for the *lac* repressor protein, a T7 promoter which is specific to T7 RNA polymerase, a transcription termination sequence, a *lac* operator which can block transcription, a polylinker for cloning, an f1 origin of replication, an ampicillin or kanamycin resistance gene, and a ColE1 origin of replication. pET vectors for use in the methods of the present invention comprise, but are not limited to, pET-21a(+), pET-24a(+), pET-28a(+), pET-29a(+), pET-30a(+), pET-41a(+), pET-44a(+), pET-21b(+), pET-24b(+), pET-26b(+), pET-28b(+), pET-29b(+), pET-30b(+), pET-42b(+),pET-44b(+). Vectors which are based on the pET-26b(+) backbone are particularly preferred. Further examples of suitable vectors are described, e.g. in "Cloning Vectors" (Pouwels et al. (eds.) Elsevier, Amsterdam New York Oxford, 1985).

An expression vector for use in bacterial cells may be transformed into the host cell by any suitable method. For example, an expression vector for use in *E. coli* may be introduced into the host cell, e.g. by electroporation or by chemical methods such as calcium phosphate-mediated transformation, as described in Maniatis et al. 1982, Molecular Cloning, A laboratory Manual, Cold Spring Harbor Laboratory.

The host cells and methods of the invention are useful for the recombinant expression of high amounts of soluble interferon proteins. The type of interferon is not particularly limited. Generally, the interferon proteins selected for expression in the host cells of the invention can be of human or non-human origin. Interferon polypeptides of non-human origin comprise, for example, bovine, equine, porcine, and murine interferons, as well as the respective proteins from dogs, cats, rabbits and sheep. Other types of non-human interferons include those that can be found in primates, such as chimpanzees and gorillas. In a preferred embodiment, the interferon proteins selected for expression in the host cells of the invention are human interferons, i.e. interferons that occur naturally in humans, including allelic variants.

The interferon protein can belong to any interferon subclass that is known in the art. In particular, the interferon protein to be expressed in the host cell of the invention can be an alpha-interferon (IFN-α), a beta-interferon (IFN-β) or a gamma-interferon (IFN-γ). In a particularly preferred embodiment, the interferon protein to be expressed is human IFN-α, IFN-β or IFN-γ or a biologically active fragment thereof. The methods of the invention can further be useful for expressing biologically active variants or fragments of IFN-α, IFN-β or IFN-γ proteins as described below.

In one embodiment of the invention, the IFN expressed in the host cells of the invention is the mature native human IFN-α protein. However, the present invention also encompasses the expression of biologically active IFN-α variants and fragments as described elsewhere herein. Human IFN-α occurs as a family of at least 25 highly homologous proteins of 166 amino acids that are encoded by distinct genes. The IFN-α proteins inhibit viral replication and cellular proliferation and modulate certain immune responses. Recombinant IFN-α is currently used in the treatment of hepatitis B and hepatitis C and also in the treatment of leukemia, malignant melanoma, and AIDS-related Kaposi's sarcoma. Preferred IFN-α for expression according to the method of the invention include IFN-α2a, IFN-α2b, and IFN-α2c. The amino acid sequence of mature native human IFN-α2a is depicted in SEQ ID NO:1.

In another embodiment, the IFN expressed in the host cells of the invention is the mature native human IFN-β protein or a biologically active IFN-β variant or fragment as described below. Human IFN-β is a glycoprotein having a molecular weight of about 20 kd. Like IFN-α, it shows a potent antiviral activity and is known to inhibit cell proliferation. The mature form of human IFN-β has 166 amino acids. Mature human IFN-β is shown in SEQ ID NO:2. IFN-β was found to bind to the same receptors as IFN-α.

In still another embodiment of the invention, the IFN expressed in the host cells of the invention is the mature native human IFN-γ protein. However, the present invention also encompasses the expression of biologically active IFN-γ variants and fragments as described elsewhere herein. IFN-γ is a glycoprotein that exerts several regulatory functions in the immune system. For example, the release of IFN-γ promotes the differentiation of B cells, and stimulates the production of TNF-α. Further, IFN-γ was found to activate epithelial and endothelial cells, fibroblasts and macrophages so as to eradicate intracellular pathogens. The effects of IFN-γ are mediated by binding of the regulatory protein to distinct forms of receptors at the surface of IFN-γ responsive cells. In its mature form, monomeric human IFN-γ encompasses 143 amino acids and has a molecular weight of about 17 kd. The amino acid sequence of mature native human IFN-γ is depicted in SEQ ID NO:3.

It is of course also possible to use the host cells and methods of the invention for the production of interferon variants which differ to some extent from the interferon proteins mentioned above, and in particular those set forth in SEQ ID NOs:1-3. The variants will typically be biologically active variants, i.e. they retain at least part of the biological activity of the naturally occurring interferon, e.g. the ability to bind to their respective receptor molecules. Suitable assays for determining the binding of a variant to interferon receptor type I are described, for example, in US Patent No. 5,766,864. Alternatively, the biological activity of an interferon variant may be determined by measuring its anti-proliferative or anti-viral activities, as described, e.g., in US Patents 5,770,191 and 5,690,925. Preferably, the variant interferon retains a considerable part of the biological activity of the interferon protein from which it derives, e.g., at least about 50% of the activity, more preferably, 60%, 70%, 80%, 90%, 95%, or more.

Variants include naturally occurring and recombinantly produced proteins which differ from the original reference amino acid sequence by one or more amino acid substitutions, deletions or additions. For example, a variant of human interferon α2a may have an amino acid sequence which differs in 2, 3, 4, 5, 6, or up to 10, 20, 30 or more positions from the sequences of native human interferon α2a depicted in SEQ ID NO:1. It is preferred that the substitutions are conservative substitutions, i.e. substitutions of one or more amino acid residues by an amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Also considered as interferon variants are proteins which differ from the original interferon by one or more (e.g. 2, 3, 4, 5, 10, or 15) additional amino acids. These additional amino acids may be present within the amino acid sequence of the original interferon proteins (i.e. as an insertion), or they may be added to one or both termini of the protein. Basically, insertions can take place at any position provided that the addition of amino acids does not impair the capability of the protein to exert at least part of the biological activity that is exerted by the naturally occurring interferon protein. Moreover, variants also comprise those proteins in which, compared to the original protein, one or more amino acids are lacking. Such deletions may affect any amino acid position provided that they do not impair the capability of the variant to exert at least part of the biological activity that is exerted by the naturally occurring interferon protein.

Variants of the interferon proteins also encompass proteins with structural modifications relative to the naturally occurring proteins, such as modified amino acids. According to the invention, modified amino acids are amino acids which have been modified either by natural processes, such as processing or post-translational modifications, or by chemical modification processes. Amino acid modifications that can occur in the interferon variants produced according to the invention may comprise phosphorylation, glycosylation, acetylation, acylation, branching, ADP ribosylation, crosslinking, disulfide bridge formation, formylation, hydroxylation, carboxylation, methylation, demethylation, amidation, cyclization and/or covalent or non-covalent bonding to phosphatidylinositol, flavine derivatives, lipoteichonic acids, fatty acids or lipids. Such modifications have been extensively described in the literature, e.g., in Proteins: Structure and Molecular Properties, T. Creighton, 2nd edition, W. H. Freeman and Company, New York (1993).

The interferon variants referred to herein preferably exhibit a significant amino acid sequence identity compared to the original interferon protein. Preferably, the amino acid identity amounts to about 60%, 65%, 70%, 75%, 80%, 85%, 90%, 95%, and more preferably to more than 96%, 97%, 98%, or 99%. Methods and computer programs for determining amino acid homology are well known in the art. For the purpose of the present invention, sequence identity is determined using the Smith-Waterman homology search algorithm (Smith and Waterman, Adv. Appl. Math. (1981), 2:482-489).

Biologically active variants of human interferon proteins have been described in the art. Human IFN-α variants can be found, for example, in US Patent 5,676,942, which also provides information regarding the residues and regions of the IFN-α protein that can be altered without the loss of biological activity. Variants of human IFN-γ are described, e.g., in US Patents 5,690,925 and 6,046,034.

The host cells and methods provided by the present invention may also be useful for the expression of biologically active fragments of the full-length interferon proteins or their variants as defined above. As used herein, a fragment of an interferon protein is a protein, polypeptide or peptide which differs from the corresponding interferon protein by the lack of one or more amino acids at the N-terminus and/or the C-terminus, wherein at least a part of the biological activity of the reference interferon molecule, e.g., its receptor binding properties, or its anti-viral or anti-proliferative activity, is retained. It is preferred that the biologically active fragments of naturally occurring interferon proteins or the fragments of the corresponding interferon variants will retain at least about 50%, at least about 75%, preferably at least about 80%, at least about 85%, at least about 90% or even up to at least about 99% of the biological activity of the reference interferon.

Biologically active fragments of human interferons have been described earlier. For example, fragments of IFN-γ are known from US Patent 5,770,191 which discloses fragments comprising amino acids 95-134 of mature full-length human IFN-γ. The fragments exert the biological activity of the mature IFN-γ. EP 0 306 870 discloses fragments of human IFN-γ having a deletion at the C-terminal amino acids 7-11. The fragments show significantly increased activity compared to full-length human IFN-γ.

When cloning a polynucleotide sequence which encodes one of the above interferon proteins or a biologically active variant or fragment thereof into a bacterial expression vector, it should be considered that it may be helpful to modify the interferon-encoding polynucleotide sequence in order to adapt it to prokaryotic expression. Methods for optimizing the codon-usage of a polynucleotide sequence to be expressed to render it amenable for expression in bacterial host cells, such as *E. coli,* are well known in the art and are described, e.g. in Maertens et al. (2010), Protein Science, 19: 1312-1326.

Briefly, the polynucleotide sequence which encodes the interferon protein to be expressed in the host cells is manipulated by adapting the codon usage to the codon bias of *E. coli.* Regions of very high (> 80%) and very low (< 30%) GC content were avoided where possible. Also, cis-acting sequence motifs, such as internal TATA-boxes, chi-sites and ribosomal entry sites, repeated sequences, RNA secondary structures and AT-rich or GC-rich sequence stretches were avoided. The optimization of gene sequences, such as human gene sequences, for expression in bacterial host cells like *E. coli* is also carried out by providers like Geneart (Regensburg, Germany).

A polynucleotide sequence encoding human IFN-α2a that has been optimized for expression in *E. coli* is provided in SEQ ID NO:4.

The invention also relates to a method for the recombinant expression of a soluble interferon which makes use of a host cell described in more detail above. The method comprises the steps of
(a) providing a host cell that comprises a functional endogenous *trxB* gene, an inactivated endogenous *gor* gene, and a polynucleotide sequence encoding an exogenous interferon protein;
(b) culturing the host cell under conditions that allow the expression of the interferon protein; and
(c) obtaining the soluble interferon protein from the host cell.

The host cell preferably comprises a copy of a bacteriophage RNA polymerase integrated in its genome, e.g. the T7 RNA polymerase, and is transformed with an episomal expression construct, e.g. an expression vector, comprising a polynucleotide sequence which codes for an exogenous interferon protein. The host used in the method for the recombinant expression of a soluble interferon can generally be any type of eukaryotic or prokaryotic cell, as discussed elsewhere herein. Preferably, the host cell is a bacterial cell, more preferably an *E. coli* cell.

According to the method provided by the present invention, the host cells are cultured under conditions that allow the expression of the interferon protein. The specific conditions will depend on the choice of the specific expression system and will also depend on factors like the nature of the host cell, the nature of the inducible promoter, and the like. The skilled person will readily be able to select and optimize the conditions which are required for the effective expression of the interferon protein in the cell culture without any inventive effort.

For example, culturing of *E. coli* host cells expressing an interferon protein can be performed in accordance with standard fermentation methods. Typically, *E. coli* cells can be cultured in a batch or fed-batch process. Culturing methods for bacterial host cells are generally described in Encyclopedia of Bioprocess Technology: Fermentation, Biocatalysis, and Bio-separation, Volumes 1-5, Flickinger, M.C., Drew, S.W. (eds.), 1999 John Wiley & Sons. Typical temperature conditions will be in the range of 20°C to 42°C, more commonly 30°C to 40°C, and preferably 35°C to 38°C. The culture medium will typically have a pH of between 6.5 and 9.0, more typically 7.0 to 8.0, e.g. 7.5. Fermentation of the culture will be continued for a time period ranging from several hours to several days. In particular, if the culturing is performed as a batch process, the culturing time normally ranges from about 12 hours to about 36 hours. When using a continuous process, fermentation times might be up to 21 days or longer.

In the last step of the method, the soluble interferon protein is obtained from the host cell. Typically, the protein is isolated from the cytosol of the host cells. The cells can be disrupted, e.g., by a French press, by repeating cycles of freezing and thawing, or by sonication. Cellular debris can be easily removed from the protein fraction by centrifugation. The soluble interferon proteins can then be purified from other protein and non-protein components by standard chromatography methods, including, for example, size exclusion chromatography and/or ion exchange chromatography. Other methods which are typically applied in protein purification, such as ammonium sulphate precipitation and filtration and/or dialysis may also be used. The interferon protein expressed by the host cell of the invention may also be purified by affinity purification using, e.g., monoclonal or polyclonal anti-interferon antibodies.

To simplify purification, the interferon produced according to the method of the invention may be expressed as a fusion protein which comprises an affinity tag that facilitates binding of the fusion protein via a compound exhibiting binding affinity to the tag. For example, the affinity tag may be a polyhistidine tag comprising 6-12 histidine residues which specifically interacts with a nickel ion chelate matrix. Alternatively, the tag may be glutathione-S-transferase allowing the purification on a glutathione matrix. Further affinity tags are well-known in the art. Non-limiting examples for pairs of affinity tag and affinity ligand include maltose-binding-protein (MBP) and maltose; avidin and biotin; Streptag and streptavidin; myc epitope and antibody. Where the affinity tag is a peptide or polypeptide, such tag may conveniently be expressed together with the interferon protein as a single expression product. Where the affinity tag is not of proteinaceous origin, it may be attached by chemical coupling reactions. For example, biotin may be chemically coupled to the fusion protein.

Preferably, at least 50% of the interferon produced according to the method of the invention is active interferon. More preferably, at least 60%, at least 70%, at least 80%, at least 90%, or at least 95% of the interferon obtained from the method of the invention is active interferon.

### BRIEF DESCRIPTION OF THE FIGURES

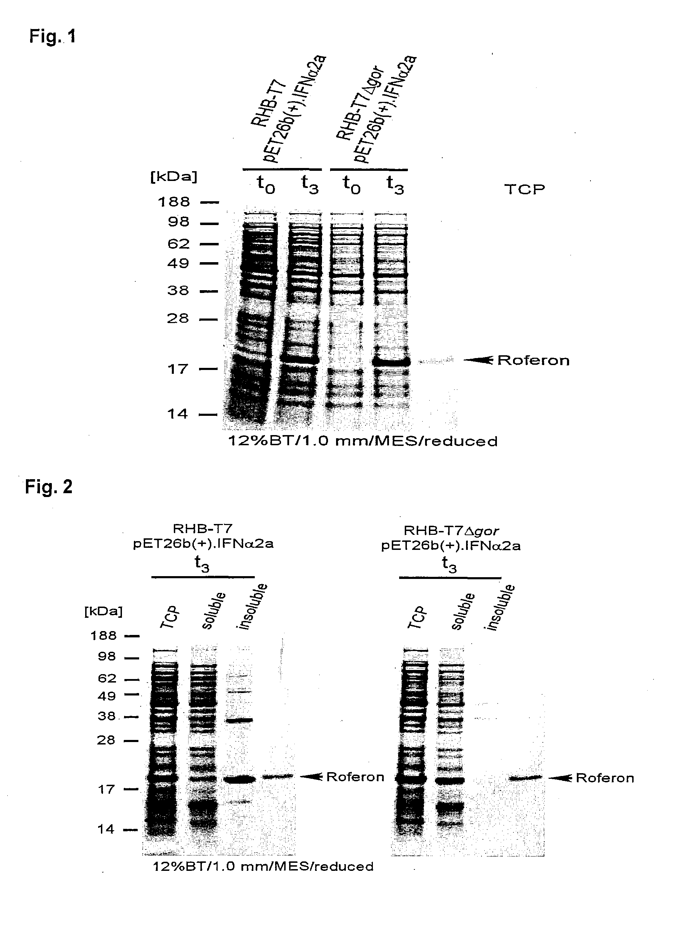

Fig. 1 shows a comparison of IFNα2a protein levels in RHB-T7/pET26b(+).IFNα2a and RHB-T7Δ*gor*/pET26b(+). IFNα2a as determined in the SDS-PAGE before (t0) and 3 hours after IPTG induction (t3). It can be seen that both clones express the IFNα2a in similar amounts.
Fig. 2 shows the results from an SDS-PAGE-analysis of soluble and insoluble protein fractions of RHB-T7/pET26b(+).IFNα2a and RHB-T7Δ*gor*/pET26b(+).IFNα2a 3 hours after IPTG induction (t3). While the RHB-T7/pET26b(+).IFNα2a culture produced mostly insoluble interferon (third lane in the left panel), RHB-T7Δ-*gor*/pET26b(+).IFNα2a showed predominantly soluble protein product (second lane in the right panel).

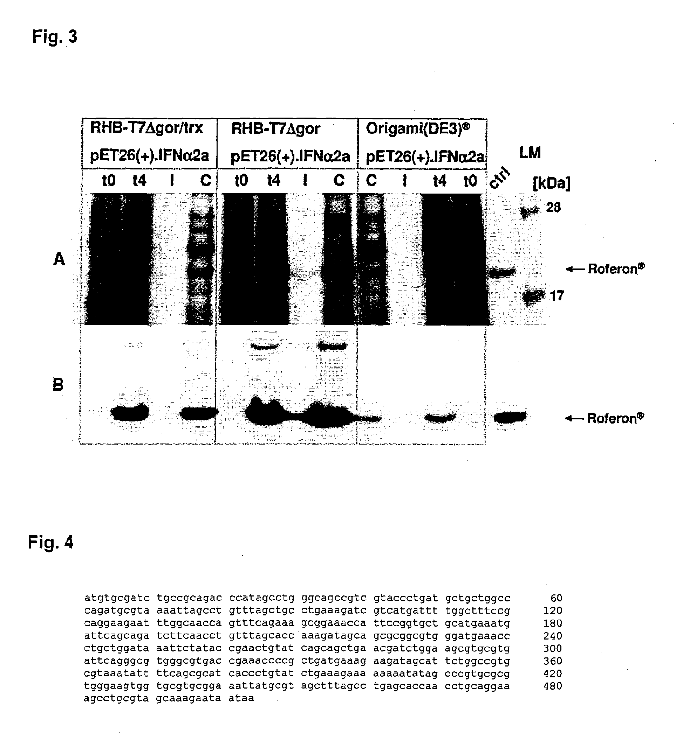

Fig. 3 shows the comparison of IFNα2a expression in the strains RHB-T7Δ*gor*, RHB-T7Δ*gor*/*trx* and Origami(DE3), all of which were previously transformed with the expression vector pET26b(+).IFNα2a. Panel A shows the results after SDS-PAGE and coomassie staining. Panel B shows the results of immunoblotting and chemoluminescence detection. t0: non-induced; t4: induced for 4h with IPTG; I: inclusion body or insoluble fraction; C: cytosol or soluble fraction; ctrl: recombinant IFNα2a (Roferon®); LM: molecular weight marker Invitrogen).
Fig. 4 shows the nucleotide sequence coding for human IFNα2a which has been optimized in terms of the codon usage for recombinant expression in an *E. coli* host.

### EXAMPLES

The invention will be illustrated by the following Examples which are given by way of example only. Specifically, the Examples describe the preparation of an *E. coli* host cell with an exogenous T7 RNA Polymerase gene and an endogenous *gor* gene that has been inactivated by homologous recombination. The Examples further describe the recombinant expression of human interferon alpha, beta and gamma in said *E. coli* host cell.

### Example 1: Preparation of a T7-controlled E. coli strain

An *E. coli* producer strain containing a genomic T7 RNA polymerase gene under control of the *lacI* repressor was prepared using the Red/ET technology for homologous recombination (Gene Bridges, Heidelberg, Germany) starting from the BL21 strain. The preparation of the producer strain included the following steps:

### a) Amplification and cloning of a T7 gene cassette

Genomic DNA of *E. coli* BL21-DE3 (Novagen) was prepared by use of a standard kit (Qiagen, Hilden, Germany). The 4.44 kb la-cUV5-T7 gene cassette of BL21-DE3 was amplified by PCR reaction. The DNA cassette contained the *lac*I gene, the regulatory domains of the lac operon, a partial lacZ gene, and the *E*. *coli* phage T7 gene 1 which encodes the T7 RNA polymerase. The PCR reaction mixtures contained KOD Hot Start DNA polymerase buffer, 2 mM MgSO₄, 200 µM dNTPs, 1 U KOD Hot Start DNA polymerase (Novagen), 10 µM forward primer (λint1), 10 µM reverse primer (λint2) and 50 ng DNA-template. A Mastercycler gradient cycler (Eppendorf) was used with the following settings: denaturation for 2 min at 98°C, followed by 34 cycles with 30 sec 98°C, 30 sec 60.5°C annealing temperature, and 2 min of elongation at 72°C. Final elongation was done for 7 min at 72°C. The following primers were used:
λint1: GTCCGACTTATGCCCGAGAAGATGTTGAGCAAACTTATCGCTTATC (SEQ ID NO:6);
λint2: TGCAAAGAGATTCTTGGCGGAGAAACCATAATTGCATCTACTCG (SEQ ID NO:7);

The resulting PCR product was subsequently used as template for a re-amplification using the same PCR conditions, designed to generate restriction sites for *Pst*I and *Sal*I to the T7 cassette. For the re-amplification PCR, the following primers were used:
PstI-forward: GGAAGCGGGCCTGCAGCGGACACCATCGAATGGCGC (SEQ ID NO:8); and
SalI-reverse: GGGGGGGGGGGTCGACGGAGTCGTATTGATTTGGCG (SEQ ID NO:9).

Following restriction analysis and elution of the resulting fragment from the agarose gel using the QIAquick gel extraction kit (Qiagen, Hilden, Germany) a ligation reaction (Rapid DNA ligation kit, Roche) was performed with a *Pst*I/*Sal*I digested pIBD5 plasmid (which includes a T5 promoter, inducible by IPTG). 100 µl of *E. coli* K12 DH10B cells were transformed by electroporation as described elsewhere herein, plated on LB agar plates containing kanamycin (Kan) and cultured at 37°C over night. One clone on the plates was cultivated for plasmid-DNA preparation (Qiaprep Spin Miniprep kit; Qiagen), digested with *Pst*I and *Sal*I and the correct insertion of the T7-RNA Polymerase (RNAP) cassette was verified by Agarose gel electrophoresis. The newly constructed vector was named pSI. The T7 RNAP gene in pSI was confirmed by sequencing (GATC Biotech AG). The approved pSI plasmid was used as a template to generate linear DNA for the recombination step, according to the technical protocol of the manufacturer (Gene Bridges).

### b) Preparing linear DNA for homologous recombination

To generate linear DNA for recombination, the T7 cassette and the kanamycin resistance gene from pSI were amplified by PCR, adding homology arms on either side that are DNA stretches homologous to the DNA of the desired genomic integration locus, i.e. recA locus of *E. coli.* The PCR reaction mixtures contained KOD Hot Start DNA polymerase buffer, 2 mM MgSO₄, 200 µM dNTPs, 1 U KOD Hot Start DNA polymerase (Novagen), 10 µM forward primer (5'harecA), 10 µM reverse primer (3'harecA) and 50 ng DNA-template. A Mastercycler gradient (Eppendorf) was used with the following settings: denaturation for 2 min at 98°C, followed by 35 cycles with 30 sec 98°C, 30 sec 65.5°C annealing temperature, and 2 min of elongation at 72°C. Final elongation was done for 7 min at 72°C. The following primers were used:
5'harecA:
3'harecA:

PCR products were purified with the QIAquick PCR purification kit (Qiagen) and digested with 10 units of *Dpn*I for 30 min at 37°C.

### c) Transforming competent E. coli cells

*E. coli* cells were transformed with the pRed/ET plasmid by electroporation of BL21 cells (Novagen). Briefly, 1 µl pRed/ET plasmid DNA was added to a 50 µl aliquot of electrocompetent cells, transferred to a 1 mm electroporation cuvette and electroporated with 1350 Volt using the Electroporator #2510 (Eppendorf). The time constant should be between 5.0 and 5.4 msec. 1 ml of pre-chilled LB medium (1% soy-peptone, 0.5% yeast extract, 1 % NaCl, pH 7.0) was added, the sample was transferred to a 1.5 ml tube, and incubated in a thermocycler for 1 h at 30°C and 800 rpm. 100 µl of the cell suspension were plated on LB agar plus tetracycline (3 µg/ml) and plates were incubated over night at 30°C. A single colony was picked from the plates of the pRed/ET electroporation and incubated over night in 1 ml LB medium plus tetracycline at 30°C, shaking at 800 rpm. 30 µl of that culture were transferred to two tubes containing 1.4 ml of LB medium plus antibiotic. Cells were grown for approximately 2 h at 30°C until they reached an optical density (OD) of 0.3. One culture was induced by addition of 50 µl 10% L-arabinose while the other remained non-induced and served as negative control. Both cultures were incubated at 37°C, 800 rpm for about 1 hour. In order to produce competent cells, both cultures were centrifuged at 4°C, 13000 rpm, washed twice with 1 ml of pre-chilled 10% glycerol and resuspended in about 50 µl of 10% glycerol.

These cultures were then transformed with the linear DNA (see above) that contained the T7 and the kanamycin resistance gene from pSI as well as the homology arms for recombination. 400 to 800 ng of linear PCR fragment were added to both the induced and non-induced cells and the suspension was electroporated with 1350 Volt. Care was taken that the volume of the DNA sample did not exceed 4 µl. 1 ml ice-cold LB medium was added and cells were incubated for 3 hours at 37°C, 800 rpm. Following centrifugation cells were resuspended in 100 µl of LB medium, plated on LB agar plus the selected antibiotic in the recommended concentration and incubated over night at 37°C. Two clones were identified. Successful recombination of the cassette into the recA locus in BL21 in these clones was confirmed by colony PCR. Sequence analysis of the whole integrated sequence (lacI, ΔlacZ, and T7 RNA polymerase gene) revealed absolute identity with the original sequence of this region in BL21-DE3. The new strain was designated RHB-T7 Kan^{r}.

### d) Removing kanamycin resistance gene

As described above, the new strain RHB-T7 Kan^{r} harbours a kanamycin resistance gene in the bacterial genome. Since the cells generated by the recombination events were intended to be used with pET vectors, further optimisation was required to avoid a double resistance (genomic and episomal resistance). Thus, the genomic kanamycin resistance gene was disrupted by inserting into the kanamycin locus a FRT-flanked chloramphenicol resistance cassette via homologous recombination. Subsequently, another recombination event controlled by the flp protein led to a deletion of about 60% of the kanamycin resistance gene. This knockout mutant allows selection of pET transformants due to the episomal kanamycin resistance gene (Kan).

The kanamycin resistance gene was removed from the genome as follows: A linear PCR product of the FRT-flanked chloramphenicol resistance cassette with 5' and 3' DNA stretches homologous to parts of the kanamycin resistance gene was integrated into the genomic kanamycin locus of RHB-T7 Kan^{r} by homologous recombination. For this purpose, the Red/ET technology for homologous recombination (Gene Bridges, Heidelberg, Germany) was used. Briefly, electrocompetent RHB-T7 Kan^{r} cells were transformed with the pRed/ET plasmid according to the protocol of the manufacturer (see above). Selection occurred on tetracycline plates and positive clones were transformed with a linear PCR fragment containing 5' and 3' homology arms of the Kan^{r} gene sequence and the FRT-flanked Cm resistance gene. Transformants were checked by PCR analysis to verify the modified genome. Seven single clones were picked and analysed by colony PCR for correct insertion into the kanamycin locus of RHB-T7 Kan^{r}. All seven clones analysed were positive. One of these clones was transformed with the pFLP707 plasmid supplied by Gene Bridges. This plasmid carries the flp recombinase gene under transcriptional control of a thermo sensitive promoter, a thermo sensitive origin of replication, and the tetracycline resistance gene for selection of transformants.

A single clone of this transformation was cultivated at 30°C. A shift in temperature to 37°C induced both the expression of the flp recombinase protein and the loss of pFLP707 plasmid including the tetracycline resistance. The cells were plated on LB agar and single clones were analysed by colony PCR for positive flp recombination events.

Three colonies carrying the FRT-flanked chloramphenicol resistance cassette were cultivated in a microfuge tube containing 1.0 ml LB medium and 15 µg/ml chloramphenicol. A hole was punctured in the lid for air supply. The culture was incubated overnight at 37°C with shaking at 800 rpm. A microfuge tube with 1.4 ml LB medium and 15 µg chloramphenicol was inoculated with 30 µl of the fresh overnight culture, incubated for 2-3 h at 37°C with shaking at 900 rpm. Cells were centrifuged for 30 sec at 11.000 rpm in a cooled microfuge and the supernatant was discarded by tipping. The pellet was resuspended in 1 ml chilled ddH₂O. The centrifugation and resuspension was repeated once. Following centrifugation the supernatant was tipped out once more and 20-30 µl were normally left in the tube with the pellet. Cells were resuspended and permanently kept on ice. Electroporation was performed with 1 µl pFLP707 DNA. Subsequent to overnight incubation on tetracycline plates at 30°C 1. ml of LB medium was inoculated with a single colony and incubated at 30°C for 2-3h. The temperature was changed to 37°C and the culture was incubated overnight. One drop of the cell suspension was plated on LB agar. Following incubation overnight at 37°C single colonies were analysed by PCR for the successful removal of the selection marker chloramphenicol. Additionally, colonies were replica-plated on both LB agar and LB agar conditioned with 15 µg/ml chloramphenicol for selection of positive clones. Two positive clones were identified by loss of chloramphenicol resistance. The clones were confirmed by sequence analysis of the respective genomic region. The new strain was named RHB-T7.

### Example 2: Disruption of the gor gene

For the preparation of electrocompetent cells, 20 ml LB medium were inoculated with an overnight culture of RHB-T7 to reach a start OD₆₀₀ of around 0.1. Cells were grown at 37°C up to an OD₆₀₀ of approximately 0.6. The culture was centrifuged at 4°C for 10 min and 4600 rpm. The supernatant was discarded while the pellet was resuspended in 14 ml of ice-cooled 10% glycerol. Subsequent to centrifugation (same conditions as above) the washing step was repeated. Finally the pellet was resuspended in 700 µl 10% glycerol and 50 µl aliquots of the cell suspension were immediately frozen in liquid nitrogen.

1 µl pRed/ET plasmid DNA was added to a 50 µl aliquot of electrocompetent cells. After mixing, the sample was kept on ice. The transformation sample was transferred to a 1 mm electroporation cuvette and electroporated at 1350 Volt using the Electroporator #2510 from Eppendorf with a time constant between 5.0 and 5.4 msec. 1 ml of pre-chilled LB medium was added, the sample was transferred to a 1.5 ml tube, and incubated in a thermocycler for 1 h at 30°C and 800 rpm. 100 µl of the cell suspension were plated on LB agar plus tetracycline (3 µg/ml) and plates were incubated over night at 30°C.

A single colony was picked from the plates of the pRed/ET plasmid electroporation and incubated over night in 1 ml LB medium plus tetracycline at 30°C, shaking at 800 rpm. 30 µl of that culture were transferred to two tubes containing 1.4 ml of LB medium plus antibiotic. Cells were grown for approximately 2 h at 30°C until they reached an OD of around 0.3. One culture was induced by addition of 50 µl 10% L-arabinose while the other remained non-induced and served as negative control. Both cultures were incubated at 37°C, 800 rpm for about an hour. In order to produce competent cells, both cultures were centrifuged at 4°C, 13.000 rpm, washed twice with 1 ml of pre-chilled 10% glycerol and resuspended in about 50 µl of 10% glycerol. These cells were used for transformation with a linear PCR fragment containing 5' and 3' homology arms of the *gor* gene sequence.

The FRT-Cm-FRT resistance cassette as supplied by Gene Bridges (Heidelberg, Germany) was used as a template for the disruption of the chromosomal *gor* gene in the RHB-T7 obtained from Example 1. PCR reactions were performed using a Mastercycler gradient from Eppendorf with the following settings: initial denaturation for 2 min at 98°C, followed by 34 cycles with 30 sec 98°C, 30 sec 60°C annealing temperature, and 1.5 min of elongation at 72°C. Final elongation was done for 7 min at 72°C and storage occurred at 4°C. The KOD Hot Start DNA Polymerase (Novagen) was used, and the primers were as follows:
5'hagor:
3'hagor:

In this way, a 1.5 kb PCR product containing the chloramphenicol resistance gene flanked by the FRT sites and the 50 bp *gor* homology arms was created. PCR products were purified with the QIAquick PCR purification kit (Qiagen). 400 to 800 ng of the linear PCR fragment containing 5' and 3' homology arms of the *gor* gene sequence and the FRT-flanked Cm resistance gene were added to both the induced and non-induced cells prepared above and the suspension was electroporated with 1350 Volt.

The purified PCR product was used to electroporate RHB-T7 transformed with the vector pRed/ET (Gene-Bridges) and thus prepared for homologous recombination. The recombination event resulted in the precise integration of the resistance marker cassette into the *gor* locus of the RHB-T7 genome. Successful disruption of the *gor* gene was confirmed by PCR. Care was taken that the volume of the DNA sample did not exceed 4 µl. 1 ml ice-cold LB medium was added and cells were incubated for 3 hours at 37°C, 800 rpm. Following centrifugation cells were resuspended in 100 µl of LB medium, plated on LB agar with chloramphenicol and incubated over night at 37°C. Successful recombination into the genome was checked by colony PCR. These PCR reactions as well as the results from replica plating experiments demonstrated that the resistance marker cassette was successfully integrated. Positive clones were transformed with pFLP707 plasmid (Gene Bridges) as described in Example 1 in order to initiate the elimination of the resistance marker. Loss of the resistance marker was confirmed by PCR and replica plating.

Integration and flipping resulted in the disruption of the *gor* open reading frame and thereby created a *gor* deficient mutant strain. This was verified by sequencing analysis. The *gor* open reading frame in RHB-T7 gor⁻ produces a truncated protein with the translation stop at TAA at position 364-366 (triplet 116 of the wild type protein). The T3 promoter sequence, Flipase Recombination Target site (FRT) and the T7 promoter sequence remain in the chromosome after the flipping event and thereby evoke the disruption of the reading frame. The new strain was named RHB-T7Δ*gor*.

### Example 3: Construction of pET26b(+).IFNα2a

The construction of the pET26b(+)IFNα2a expression vector was performed after agarose gel electrophoresis and gel elution of the Nde/Sal-digested pET26b(+) (Invitrogen) plasmid fragment as well as the Nde/Sal-digested and codon-optimized IFNα2a insert fragment (pMA0813119, GeneArt). Based on these two isolated DNA fragments (Qiagen minelute gel extraction kit) a ligation reaction was performed using the Roche Rapid DNA Ligation kit. After transformation of chemically competent *E. coli* DH10B cells with an aliquot of the ligation mixture, individual colonies were picked from kanamycin-containing LB-plates and cultured overnight at 37°C in liquid LB-medium containing 50 µg/ml of the selection marker. Afterwards, plasmid DNA was isolated from each clone, followed by a digest using the Nde/Sal restriction enzymes. The digest resulted in a Nde/Sal-fragment of about 550 bp in two clones with a vector fragment of the correct size of >5000 bp. In order to confirm that the clones comprise the correct expression vector pET26b(+)IFNα2a, clone 1 was subjected to a more specific cleavage using different restriction enzymes, such as PstI, BgIII, BspHI and SspI. The result of the restriction cleavage and agarose gel electrophoresis confirmed the correct number and sizes of DNA-fragments. Thus, the expression vector pET26b(+)IFNα2a was transformed into competent RHB-T7Δgor cells and resulted in kanamycin resistant colonies from which five were randomly chosen for inoculation of 3 mL kanamycin-containing liquid cultures and storage of corresponding cryostocks at -80°C.

### Example 4: Expression analysis of IFNα2a

*E. coli* cells from the strains RHB-T7 and RHB-T7Δgor described above were cultured to the mid-log growth phase. Cells were centrifuged (1 min/16.100 x g/0°C) and resuspended in ice-cold 10% glycerol and centrifuged again (1 min/16.100 x g/0°C). This washing step was repeated once. After the second washing cells were resuspended in 50 µL ice-cold 10% glycerol and the suspension was transferred into a pre-chilled electroporation cuvette (Gap: 2 mm). An aliquot of the pET26b(+).IFNα2a expression vector was added to the cuvette and electroporation was carried out with an Electroporator 2510 (Eppendorf AG, Germany) applying 1350 Volts (V). After electroporation, 1 mL of pre-chilled LB medium was added and the cell suspension was transferred to a fresh tube. Following an incubation of one hour at 37°C with agitation (250 rpm) an aliquot of 50 µL was spread out on a LB agar plate, supplemented with 50 µg/mL kanamycin and incubated overnight at 37°C. Single colonies from transformation plates were picked and used for inoculation of 3 mL cultures (LB medium, supplemented with 50 µg/mL kanamycin). After overnight incubation (37°C/250 rpm) primary cryo-preservation stocks were prepared as follows: a 1 mL sample of culture was mixed with 0.5 mL sterile 30% ice-cold glycerol in a cryo tube (final: 10% glycerol). After shock-freezing in liquid nitrogen (LN2) the tubes were stored at -80°C.

The clones obtained by transformation of RHB-T7 and RHB-T7Δgor with the pET26b(+).IFNα2a expression vector were designated RHB-T7/pET26b(+).IFNα2a and RHB-T7Δgor/pET26b(+).IFNα2a, respectively. The identity and integrity of the pET26b(+).IFNα2a expression vector in the different clones obtained by transformation was confirmed by a DNA-fragmentation analysis using the restriction enzymes BglII, BspHI, NdeI, PstI, SalI and SspI.

Cultivation of RHB-T7/pET26b(+).IFNα2a and RHB-T7Δgor/pET-26b(+).IFNα2a for IFN production was started by inoculation of 3 mL complex medium [veg.] (27.0 g/L vegetable-peptone, 14.0 g/L yeast extract, 5.0 g/L NaCl, 0.5 g/L MnSO₄*H₂O, 25.0 g/L (w/v) Glycerol, 8.65 g/L K₂HPO₄, 6.85 g/L KH₂PO₄; pH 7.0 ±0.1) from cryo stocks. The medium was supplemented with 50 µg/mL kanamycin. The cultures were incubated at 37°C with 250 rpm overnight. 15 mL expression cultures were initiated by using aliquots of the 3 mL overnight cultures to achieve a starting OD₆₀₀ of 0.1 in 15 mL of complex medium [veg.] without antibiotics in a baffled shaker flask at 37°C with 250 rpm. After reaching mid-log phase with an OD₆₀₀ of 0.5 to 0.8, IPTG was added (final concentration of 1 mM) and cultures were further cultivated for 3 hours. 1 mL culture samples were taken immediately before (t₀) and 3 hours after (t₃) IPTG-induction.

The 1 mL culture samples were centrifuged (1 min/16.100 x g/0°C). Cell pellets were resuspended in 4 mL Tris-EDTA-Buffer (pH 7.4), transferred into a glass-tube (inner width 22 mm) and placed in an ice-bath. Fractionation of the cells was achieved using the sonication device UP 400S (Hielscher Ultrasonic GmbH; device parameter: 400W, 24 kHz) equipped with the sonotrode H3. Technical settings: immersion depth: approx. 15 mm; capacity: 75%; cycle: 30 seconds sonication - 30 seconds off; total treatment time: 10 min. Afterwards the sample was transferred to a 15 mL Falcon tube and centrifuged (30 min/4.080 x g/4°C). The insoluble protein fraction was forming a pellet. The supernatant, containing the soluble proteins, was transferred to a fresh 15 mL Falcon tube, spiked with 600 µL 100% trichloroacetic acid (final concentration: 15%) and incubated for one hour at 4°C before centrifugation (30 min/4.080 x g/4°C). The pellet from this step represents the soluble protein fraction. Both pellets were solubilized and adjusted to an OD₆₀₀ = 10 by adding IB buffer (8 M Urea, 50 mM DTT, 100 mM Tris-HCl; pH 8.0). A standardization step to OD₆₀₀ = 10 permitted the direct comparability of relative protein amounts in different lanes of a gel run. For the estimation of protein production rates the effectively measured OD₆₀₀ of the analyzed culture had to be taken into account.

Total cell protein (TCP) and soluble and insoluble protein fractions were applied to SDS-PAGE with coomassie staining. For all SDS-PAGE gel runs pre-cast NuPAGE^{®} Novex^{®} 12% Bis-Tris gels (Invitrogen, Karlsruhe, Germany) were used in combination with the corresponding XCell SureLock Mini-Cells (Invitrogen). According to the size of recombinant INFα2a (19 kDa), a MES buffer system (Invitrogen) was deployed to achieve the best gel resolution. All samples were dissolved in 0.25 vol. LDS sample buffer (Invitrogen), substituted with 0.1 vol. Sample Reducing Agent (Invitrogen), and heated for 10 min to 95°C before loaded on a gel. For Coomassie staining the Bio-Safe Coomassie Stain system (Bio-Rad, Munich, Germany) was used according to the supplier's recommendations. Samples to be analyzed by the Electrophoresis Station (Experion, Bio-Rad Laboratories, Hercules, USA) were treated following the instruction of the manufacturer for reducing conditions (Experion Pro260 Analysis Kit Instruction Manual) and loaded on an Experion Pro260 chip (Bio-Rad). Recording and further analysis of data was carried out with Experion Software System Operation and Data Analysis Tool (Bio-Rad, Version 3.0.226.0).

Results: 3 hours after induction both RHB-T7/pET26b(+).IFNα2a and RHB-T7Δgor/pET26b(+).IFNα2a showed prominent protein bands at about 19 kDa in SDS-PAGE analysis (the expected molecular weight of human IFNα2a is approx. 18.8 kDa). The protein band was exclusively detected in IPTG-induced cultures which demonstrates that the expression in both clones is tightly controlled (see Fig. 1). Immunoblot analysis using an anti-IFNα2a antibody confirmed that the protein band observed in the SDS-PAGE was IFNα2a (data not shown). A comparison of the intensities of the protein bands in the SDS-PAGE of RHB-T7/pET26b(+).IFNα2a and RHB-T7Δ*gor*/pET26b(+).IFNα2a revealed that both clones express the interferon protein in similar amounts (see Fig. 1).

When analyzing the soluble and insoluble protein fractions of the clones, it was found that in RHB-T7/pET26b(+).IFNα2a, the recombinant IFNα2a is almost completely present in inclusion bodies. Conversely, in RHB-T7Δ*gor*/pET26b(+).IFNα2a the produced IFNα2a was nearly completely soluble (see Fig. 2).

### Example 5: Expression analysis of IFN-β and IFN-γ

In accordance with the methods described in Examples 3-4, human IFN-β and human IFN-γ were cloned into a pET26b(+) expression vector. The resulting plasmids pET26b(+).IFN-β and pET26b(+).IFN-γ were transformed into the strains RHB-T7Δ*gor* and RHB-T7.

Results: Similar to the results observed above with IFN-α2a, it could be shown that in RHB-T7, both human IFN-β and human IFN-γ were produced mainly in the form of inclusion bodies, whereas in RHB-T7Δgor, at least about 90% of the recombinant protein was expressed in soluble form (data not shown).

### Example 6: Comparative expression analysis of IFNα2a

The RHB-T7Δ*gor* strain prepared in accordance with Example 2 was then used to create a *trxB⁻*/*gor⁻* double knock-out strain. The inactivating mutation in the *trxB* gene was generated essentially as discussed above in Example 2 in connection with the *gor* gene. The primers used for inactivation of the *trxB* gene were the following:
5'hatrx:
3'hatrx:

The resulting strain was designated RHB-T7Δ*gor*/*trx*. As explained above, *trxB⁻*/*gor⁻* double knock-out strains can only grow if they exhibit a mutation in the *ahpC*.* Therefore, in the RHB-T7Δgor/trx described herein, the *ahpC** suppressor mutation was achieved by homologous recombination. No such mutation was necessary in the RHB-T7Δgor strain.

Both RHB-T7Δ*gor* and RHB-T7Δ*gor*/*trx* were transformed with 100 ng pET26b(+)IFNα2a DNA which led to strains RHB-T7Δ-*gor*/pET26b(+).IFNα2a and RHB-T7Δ*gor*/*trx*-pET26b(+).IFNα2a, respectively.

Similarly, competent Origami(DE3) cells (Novagen) were transformed with 100 ng pET26b(+)IFNα2a DNA and incubated overnight at 37°C on selective LB agar plates with either 50 µg/ml or 150 µg/ml kanamycin. 20 colonies were found on the 150 µg/ml plate, whereas ≥500 colonies grew on the 50 µg/ml plate. Plasmid DNA preparation (Qiagen miniprep kit) and subsequent restriction digest of three independent clones picked from the 150 µg/ml plate revealed that all three clones were carrying the pET26b(+).IFNα2a expression vector. One clone was chosen for the following expression experiments (Origami(DE3)/ pET26b(+)IFNα2a.

Expression analysis and further fractionation steps, SDS-PAGE of the recombinant IFN-α2a protein and immunoblot detection were performed in accordance with the methods described in examples 3-4.

Results: It was observed that in all tested strains the majority of the recombinantly expressed IFN-α2a protein was produced in the soluble fraction. Only small amounts of protein were detected in inclusion bodies. Interestingly, the amounts of soluble IFN-α2a protein were significantly higher in RHB-T7Δ*gor*-pET26b(+).IFNα2a when compared to RHB-T7Δ*gor*/trx-pET26b(+).IFNα2a or Origami(DE3)/pET26b(+).IFNα2a. The amounts detected in RHB-T7Δ*gor*/trx-pET26b(+).IFNα2a were higher than those in Origami(DE3)/pET26b(+).IFNα2a (see Fig. 3).

## Claims

1. Host cell for the recombinant expression of a mammalian interferon protein, said cell comprising:
(a) a functional endogenous trxB gene,
(b) an inactivated endogenous gor gene, and
(c) a polynucleotide sequence encoding an exogenous interferon protein, wherein inactivated means a decreased amount of functional glutathion oxidoreductase enzyme by at least 90% compared to the unmodified host cell.

2. Host cell of claim 1, wherein said polynucleotide sequence is included in an expression vector.

3. Host cell of claim 1 or 2, wherein said interferon is selected from the group consisting of interferon alpha, interferon beta, and interferon gamma or an active fragment thereof.

4. Host cell of claim 3, wherein said interferon protein is a human interferon protein or a biologically active fragment thereof.

5. Host cell of claim 4, wherein said interferon protein is human interferon α2a or a biologically active fragment thereof.

6. Host cell of any of claims 1-5, wherein said host cell is a bacterial cell.

7. Host cell of claim 6, wherein said bacterial cell is an Escherichia coli cell.

8. Host cell of claim 7, wherein said Escherichia coli cell has been derived from Escherichia coli strain BL21.

9. Host cell of any of claims 1-8, wherein said cell comprises a T7 RNA polymerase gene.

10. Host cell of claim 9, wherein said T7 RNA polymerase gene is inserted into the genome of the host cell.

11. Method for the recombinant expression of a soluble interferon in a host cell, comprising
(a) providing a host cell of any of claims 1-10;
(b) culturing the host cell under conditions that allow the expression of the interferon protein;
(c) obtaining the soluble interferon protein from the host cell.

12. Method of claim 11, wherein step (c) comprises disruption of the cells.

13. Method of claim 12, wherein step (c) further comprises purification of the protein by chromatography.

14. Use of a host cell according to any of claims 1-10 for the recombinant expression of a soluble interferon.

## Patentansprüche

1. Wirtszelle für die rekombinante Expression eines Säugetier-Interferon-Proteins, wobei die Zelle Folgendes umfasst:
(a) ein funktionsfähiges endogenes trxB-Gen,
(b) ein inaktiviertes endogenes gor-Gen, und
(c) eine Polynukleotid-Sequenz, die für ein exogenes Interferon-Protein kodiert,
wobei inaktiviert eine im Vergleich zur unmodifizierten Zelle um mindestens 90% verringerte Menge an funktionsfähigem Glutathion-Oxidoreduktase-Enzym bedeutet.

2. Wirtszelle gemäß Anspruch 1, wobei die Polynukleotid-Sequenz in einem Expressionsvektor enthalten ist.

3. Wirtszelle gemäß Anspruch 1 oder 2, wobei das Interferon ausgewählt ist aus der Gruppe bestehend aus Interferon-Alpha, Interferon-Beta und Interferon-Gamma oder einem aktiven Fragment davon.

4. Wirtszelle gemäß Anspruch 3, wobei das Interferon-Protein ein humanes Interferon-Protein oder ein biologisch aktives Fragment davon ist.

5. Wirtszelle gemäß Anspruch 4, wobei das Interferon-Protein humanes Interferon α2a oder ein biologisch aktives Fragment davon ist.

6. Wirtszelle gemäß einem der Ansprüche 1-5, wobei die Wirtszelle eine bakterielle Zelle ist.

7. Wirtszelle gemäß Anspruch 6, wobei die bakterielle Zelle eine *Escherichia coli*-Zelle ist.

8. Wirtszelle gemäß Anspruch 7, wobei die *Escherichia coli-*Zelle vom *Escherichia coli-Stamm* BL21 abgeleitet ist.

9. Wirtszelle gemäß einem der Ansprüche 1-8, wobei die Zelle ein T7-RNA-Polymerase-Gen umfasst.

10. Wirtszelle gemäß Anspruch 9, wobei das T7-RNA-Polymerase-Gen in das Genom der Wirtszelle eingebracht ist.

11. Verfahren zur rekombinanten Expression eines löslichen Interferons in einer Wirtszelle, bei dem man
(a) eine Wirtszelle gemäß einem der Ansprüche 1-10 bereitstellt;
(b) die Wirtszelle unter Bedingungen kultiviert, welche die Expression des Interferon-Proteins erlauben;
(c) das lösliche Interferon-Protein aus der Wirtszelle erhält.

12. Verfahren gemäß Anspruch 11, bei dem Schritt (c) den Aufschluss der Zellen umfasst.

13. Verfahren gemäß Anspruch 12, wobei Schritt (c) ferner die Aufreinigung des Proteins mittels Chromatographie umfasst.

14. Verwendung einer Wirtszelle gemäß einem der Ansprüche 1-10 für die rekombinante Expression eines löslichen Interferons.

## Revendications

1. Cellule hôte pour l'expression recombinante d'une protéine interféron de mammifère, laquelle cellule comprend
a) un gène trxB endogène fonctionnel,
b) un gène gor endogène inactivé,
c) et une séquence polynucléotidique codant une protéine interféron exogène,
étant entendu que le terme « inactivé » signifie que la quantité d'enzyme glutathion oxydo-réductase fonctionnelle est diminuée d'au moins 90 %, par rapport à la cellule hôte non modifiée.

2. Cellule hôte conforme à la revendication 1, dans laquelle ladite séquence polynucléotidique est incluse dans un vecteur d'expression.

3. Cellule hôte conforme à la revendication 1 ou 2, dans laquelle ledit interféron est choisi dans l'ensemble formé par l'interféron alpha, l'interféron bêta et l'interféron gamma, ou est un fragment actif de l'un d'eux.

4. Cellule hôte conforme à la revendication 3, dans laquelle ladite protéine interféron est une protéine interféron humaine ou un fragment biologiquement actif d'une telle protéine.

5. Cellule hôte conforme à la revendication 4, dans laquelle ladite protéine interféron est de l'interféron humain α2a ou un fragment biologiquement actif de cet interféron.

6. Cellule hôte conforme à l'une des revendications 1 à 5, laquelle cellule hôte est une cellule bactérienne.

7. Cellule hôte conforme à la revendication 6, ladite cellule bactérienne étant une cellule d'Escherichia coli.

8. Cellule hôte conforme à la revendication 7, ladite cellule d'Escherichia coli ayant été obtenue à partir de la souche BL21 d'Escherichia coli.

9. Cellule hôte conforme à l'une des revendications 1 à 8, laquelle cellule comporte un gène d'ARN polymérase de T7.

10. Cellule hôte conforme à la revendication 9, dans laquelle ledit gène d'ARN polymérase de T7 est inséré dans le génome de la cellule hôte.

11. Procédé d'expression recombinante d'un interféron soluble au sein d'une cellule hôte, comportant les étapes suivantes :
a) prendre une cellule hôte conforme à l'une des revendications 1 à 10;
b) cultiver cette cellule hôte dans des conditions qui permettent l'expression de la protéine interféron ;
c) et récupérer la protéine interféron soluble à partir de la cellule hôte.

12. Procédé conforme à la revendication 11, dans lequel l'étape (c) comporte la rupture des cellules.

13. Procédé conforme à la revendication 12, dans lequel l'étape (c) comporte en outre la purification de la protéine par chromatographie.

14. Utilisation d'une cellule hôte conforme à l'une des revendications 1 à 10 pour l'expression recombinante d'un interféron soluble.
